(19) Europäisches Patentamt
European Patent Office
Office européen des brevets

(11) Veröffentlichungsnummer : **0 024 612**
**B1**

(12) # EUROPÄISCHE PATENTSCHRIFT

(45) Veröffentlichungstag der Patentschrift : **05.10.83**

(21) Anmeldenummer : **80104715.0**

(22) Anmeldetag : **09.08.80**

(51) Int. Cl.³ : **C 07 C 43/29**, C 07 C 41/16, C 07 C 43/174, C 07 C 43/225, C 07 C 33/46, C 07 C 29/136, C 07 C 17/16, C 07 C 25/13

(54) **Neue 4-Fluor-3-phenoxy-benzyl-ether und Verfahren zu ihrer Herstellung und ihre Verwendung.**

(30) Priorität : **22.08.79 DE 2933985**

(43) Veröffentlichungstag der Anmeldung : **11.03.81 Patentblatt 81/10**

(45) Bekanntmachung des Hinweises auf die Patenterteilung : **05.10.83 Patentblatt 83/40**

(84) Benannte Vertragsstaaten : **BE CH DE FR GB IT LI NL**

(56) Entgegenhaltungen : **Keine**

(73) Patentinhaber : **BAYER AG**
**Zentralbereich Patente, Marken und Lizenzen**
**D-5090 Leverkusen 1, Bayerwerk (DE)**

(72) Erfinder : **Fuchs, Rainer, Dr.**
**Roeberstrasse 8**
**D-5600 Wuppertal 1 (DE)**
Erfinder : **Maurer, Fritz, Dr.**
**Roeberstrasse 8**
**D-5600 Wuppertal 1 (DE)**
Erfinder : **Priesnitz, Uwe, Dr.**
**Severinstrasse, 58**
**D-5650 Solingen 1 (DE)**
Erfinder : **Riebel, Hans-Jochem, Dr.**
**In der Beek 92**
**D-5600 Wuppertal 1 (DE)**
Erfinder : **Klauke, Erich, Dr.**
**Eichendorffweg 8**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 024 612 B1

Neue 4-Fluor-3-phenoxy-benzyl-ether und Verfahren zu ihrer Herstellung und ihre Verwendung

Die Erfindung betrifft neue 4-Fluor-3-phenoxy-benzylether, ein Verfahren zu deren Herstellung sowie ihre Verwendung zur Herstellung von 4-Fluor-3-phenoxy-benzylbromid.

Es ist bekannt, daß man 4-Fluor-3-phenoxy-benzyl-bromid, ein Zwischenprodukt für pestizid wirksame Pyrethroide, erhält, wenn man 4-Fluor-3-phenoxy-toluol mit N-Bromsuccinimid in Tetrachlorkohlenstoff unter Verwendung von Azodiisobuttersäurenitril als Katalysator umsetzt (vergleiche DE-OS 2 709 264). Diese Synthesemethode ist jedoch wegen der hohen Kosten der Reagentien und wegen der unbefriedigenden Ausbeuten für die Herstellung von 4-Fluor-3-phenoxy-benzyl-bromid im industriellen Maßstab wenig geeignet.

Die vorliegende Anmeldung betrifft :

(1) neue 4-Fluor-3-phenoxy-benzyl-ether der Formel I

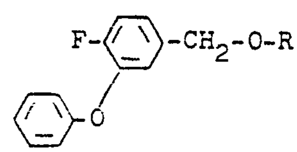

(I)

in welcher R für Phenyl oder Benzyl steht ;

(2) ein Verfahren zur Herstellung der neuen Verbindungen der Formel (I), dadurch gekennzeichnet, daß man 3-Brom-4-fluor-benzyl-ether der Formel II

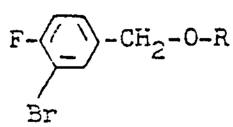

(II)

in welcher R die oben angegebene Bedeutung hat, mit Alkali- oder Erdalkali-phenolaten, gegebenenfalls in Gegenwart von Hilfsstoffen aus der Reihe der Alkali- oder Erdalkali-halogenide oder -carbonate und in Gegenwart von Kupfer oder Kupferverbindungen als Katalysatoren sowie unter Verwendung von Verdünnungsmitteln bei Temperaturen zwischen 100 und 200 °C umsetzt ;

(3) die Verwendung der neuen Verbindungen der Formel (I) als Zwischenprodukte zur Herstellung von 4-Fluor-3-phenoxy-benzyl-bromid durch Umsetzung mit Bromwasserstoff nach bekannten Etherspaltungsmethoden.

(4) 3-Brom-4-fluor-benzyl-ether der Formel II

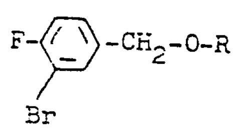

(II)

in welcher R für Phenyl oder Benzyl steht ; sind neu.

(5) Sie werden hergestellt, indem man für den Fall, daß in Formel II R für Phenyl steht, 3-Brom-4-fluorbenzylhalogenide der Formel III

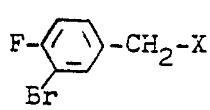

(III)

in welcher X für Chlor oder Brom steht, mit Alkali- oder Erdalkali-phenolaten gegebenenfalls in Gegenwart von Verdünnungsmitteln bei Temperaturen zwischen 0 und 150 °C umsetzt.

(6) (3-Brom-4-fluor-benzyl)-benzyl-ether (II, R = Benzyl), werden hergestellt, indem man 3-Brom-4-fluor-benzylalkohol der Formel IV

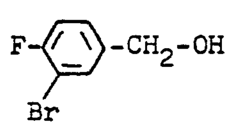

(IV)

mit Benzylhalogeniden in Gegenwart von Basen und gegebenenfalls unter Verwendung von Verdünnungsmitteln bei Temperaturen zwischen 0 und 150 °C umsetzt.

(7) 3-Brom-4-fluor-benzylhalogenide der Formel (III)

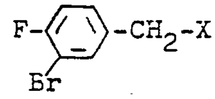

(III)

in welcher X für Chlor oder Brom steht ; sind neu.

(8) 3-Brom-4-fluor-benzylhalogenide der Formel (III) werden hergestellt, indem man 3-Brom-4-fluor-benzylalkohol der Formel (IV) (oben) mit Halogenierungsmitteln, gegebenenfalls unter Verwendung von Verdünnungsmitteln, bei Temperaturen zwischen – 10 und 100 °C umsetzt.

(9) 3-Brom-4-fluor-benzylalkohol der Formel IV ist neu.

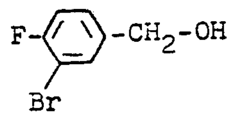

(IV)

(10) 3-Brom-4-fluor-benzylalkohol der Formel IV (oben) wird hergestellt, indem man 3-Brom-4-fluor-benzoylfluorid der Formel V

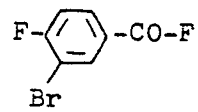

(V)

mit einem Hydridkomplex der Formel VI

$$M(M'H_4) \qquad (VI)$$

in welchem

M für Lithium, Natrium oder Kalium steht und

M' für Bor oder Aluminium steht,

gegebenenfalls in Gegenwart eines Verdünnungsmittels bei Temperaturen zwischen 0 und 50 °C umsetzt.

Mit Hilfe der neuen 4-Fluor-3-phenoxy-benzyl-ether der Formel (I) kann 4-Fluor-4-phenoxy-benzylbromid wesentlich einfacher als nach dem oben erwähnten bekannten Verfahren hergestellt werden.

Das unter (2) dargelegte Verfahren (« Verfahren (2) ») kann bei Verwendung von (4-Fluor-3-brombenzyl)-benzylether und Kaliumphenolat als Ausgangsstoffen durch folgendes Reaktionsschema skizziert werden :

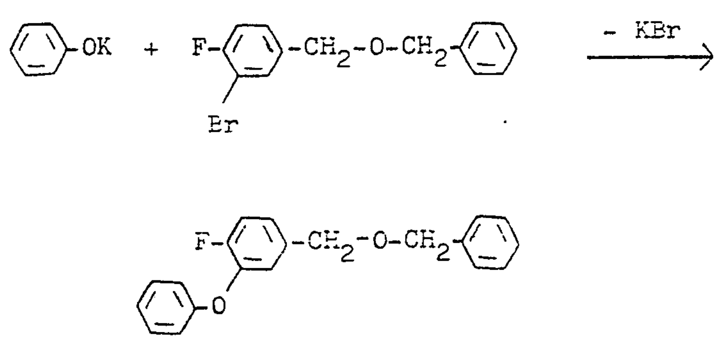


Alkali- oder Erdalkali-phenolate, welche bei Verfahren (2) als Ausgangsstoffe verwendet werden können, sind z. B. Natrium-, Kalium- und Magnesium-phenolat. Natriumphenolat wird als Ausgangsverbindung bevorzugt. Hilfsstoffe aus der Reihe der Alkali- oder Erdalkalihalogenide oder -carbonate sind z. B. Kalium- und Magnesium-chlorid sowie Kalium- und Magnesium-carbonat. Diese Hilfsstoffe werden vorzugsweise dann verwendet, wenn als Ausgangsverbindung Natriumphenolat eingesetzt wird.

Als Katalysatoren werden Kupfer oder Kupferverbindungen verwendet. Als Beispiele hierfür seien Kupfer, Kupfer(I) oxid, Kupfer(II) oxid, Kupfer(I) chlorid und Kupfer(I) bromid genannt.

Die Reaktionstemperatur wird bei Verfahren (2) zwischen 100 und 200 °C, vorzugsweise zwischen 140 und 180 °C gehalten. Das Verfahren wird gewöhnlich unter Normaldruck durchgeführt.

Auf 1 Mol 3-Brom-4-fluor-benzyl-ether der Formel (II) setzt man 1 bis 1,5 Mol, vorzugsweise 1 bis 1,2 Mol Phenolat, gegebenenfalls 10 bis 200 g eines Hilfsstoffes aus der Reihe der Alkali- oder Erdalkalihalogenide oder -carbonate sowie 0,01 bis 0,5 Mol, vorzugsweise 0,1 bis 0,5 Mol Kupferkatalysator ein.

In einer bevorzugten Variante (a) von Verfahren (2) zur Herstellung von (4-Fluor-3-phenoxy-benzyl)-benzyl-ether wird Isochinolin als Verdünnungsmittel verwendet. Zur Durchführung von Verfahrensvariante (2 a) werden die Komponenten vermischt und unter Rühren erhitzt, bis die Reaktion abgelaufen ist. Die Aufarbeitung kann auf übliche Weise durchgeführt werden. Beispielsweise wird das Reaktionsgemisch nach Abkühlen mit einem mit Wasser nicht mischbaren Lösungsmittel, wie z. B. Cyclohexan, verdünnt, filtriert, das Filtrat mit Salzsäure und Wasser gewaschen, getrocknet, filtriert und das Lösungsmittel abdestilliert. Das zurückbleibende Rohprodukt kann durch Vakuumdestillation gereinigt werden.

In einer weiteren bevorzugten Variante (b) von Verfahren (2) zur Herstellung von (4-Fluor-3-phenoxy-benzyl)-phenylether wird Bis-(2-methoxyethyl)-ether (Diglyme) als Verdünnungsmittel verwendet. Zur Durchführung von Verfahrensvariante (2 b) werden die Komponenten vermischt und unter Rühren erhitzt, bis die Reaktion abgelaufen ist. Die Aufarbeitung kann auf übliche Weise durchgeführt werden. Beispielsweise wird das Reaktionsgemisch nach Abkühlen mit einem mit Wasser nicht mischbaren Lösungsmittel, wie z. B. Toluol, verdünnt und filtriert, das Filtrat mit verdünnter Natronlauge und mit Wasser gewaschen, getrocknet, filtriert und das Lösungsmittel abdestilliert. Das zurückbleibende Rohprodukt kann durch Vakuumdestillation gereinigt werden.

Die neuen Verbindungen der Formel (I) können als Zwischenprodukte zur Herstellung von 4-Fluor-3-phenoxy-benzylbromid, welches als Zwischenprodukt für Pyrethroide bekannt ist (vergleiche DE-OS 2 709 264), verwendet werden.

Die Herstellung von 4-Fluor-3-phenoxy-benzyl-bromid durch Etherspaltung von Verbindungen der Formel (I) mit Bromwasserstoffsäure kann bei Einsatz von (4-Fluor-3-phenoxy-benzyl)-benzyl-ether durch folgendes Formelschema skizziert werden :

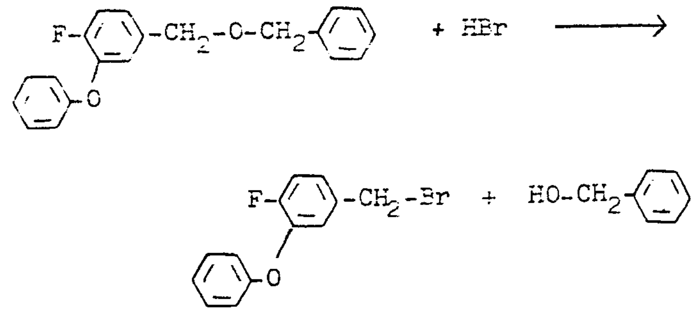

Die Etherspaltung zur Herstellung von 4-Fluor-3-phenoxybenzylbromid kann nach üblichen Methoden durchgeführt werden.

In einer bevorzugten Verfahrensweise werden die Verbindungen der Formel (I) mit wässriger Bromwasserstoffsäure und Essigsäure mehrere Stunden unter Rückfluß erhitzt. Zur Aufarbeitung wird das Reaktionsgemisch gegebenenfalls mit Wasser verdünnt und mit einem mit Wasser nicht mischbaren Lösungsmittel, wie z. B. Methylenchlorid, extrahiert. Die Extrakte werden mit verdünnter Natronlauge gewaschen und getrocknet. Dann wird das Lösungsmittel unter vermindertem Druck sorgfältig abdestilliert und das als Rückstand verbleibende Rohprodukt gegebenenfalls durch Vakuumdestillation gereinigt.

Das unter (5) dargelegte Verfahren zur Herstellung von (3-Brom-4-fluor-benzyl)-phenyl-ether (« Verfahren (5) ») kann bei Verwendung von 3-Brom-4-fluor-benzyl-chlorid und Kaliumphenolat als Ausgangsstoffen durch folgendes Formelschema skizziert werden :

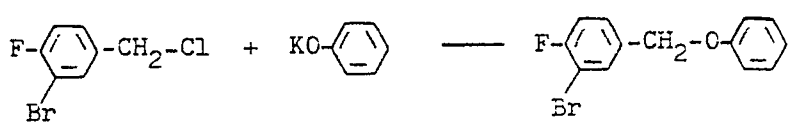

Alkali- oder Erdalkali-phenolate, welche bei Verfahren (5) als Ausgangsstoffe verwendet werden können, sind z. B. Natrium-, Kalium- und Magnesium-phenolat. Natriumphenolat wird als Ausgangsverbindung bevorzugt.

Verfahren (5) wird vorzugsweise unter Verwendung von Verdünnungsmitteln durchgeführt. Als solche kommen insbesondere aprotisch polare Lösungsmittel, wie z. B. Tetrahydrofuran, Glycoldimethylether, Acetonitril, Dimethylformamid oder Dimethylsulfoxid in Frage.

Die Reaktionstemperatur wird bei Verfahren (5) zwischen 0 und 150 °C, vorzugsweise zwischen 10 und 100 °C gehalten. Das Verfahren wird im allgemeinen bei Normaldruck durchgeführt.

Auf 1 Mol 3-Brom-4-fluor-benzyl-halogenid (III) setzt man 1 bis 1,5 Mol, vorzugsweise 1 bis 1,2 Mol Phenolat ein. In einer bevorzugten Ausführungsform von Verfahren (5) wird das Phenolat in einem Verdünnungsmittel vorgelegt und dazu unter Rühren tropfenweise das 3-Brom-4-fluor-benzyl-halogenid gegeben. Das Reaktionsgemisch wird dann, gegebenenfalls bei mäßig erhöhter Temperatur bis zum Reaktionsende gerührt. Die Aufarbeitung kann auf übliche Weise durchgeführt werden, beispielsweise indem man mit Wasser verdünnt und mit einem mit Wasser nicht mischbaren Lösungsmittel, wie z. B.

Methylenchlorid, extrahiert, die Extrakte trocknet, filtriert und einengt und den Rückstand gegebenenfalls im Vakuum destilliert.

Das unter (6) dargelegte Verfahren zur Herstellung von (3-Brom-4-fluor-benzyl)-benzyl-ether (« Verfahren (6) ») kann bei Verwendung von Benzylbromid als Reaktionskomponente durch folgendes Formelschema skizziert werden :

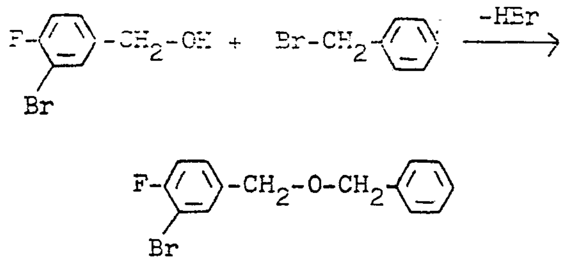


Verfahren (6) wird vorzugsweise unter Verwendung von Verdünnungsmitteln durchgeführt. Es werden die gleichen Lösungsmittel wie in Verfahren (5) bevorzugt.

Benzylhalogenide, welche bei Verfahren (6) bevorzugt eingesetzt werden, sind Benzylchlorid und Benzylbromid.

Bei Verfahren (6) werden zur Deprotonierung von Benzylalkoholen geeignete Basen verwendet. Als solche kommen Alkoholate, wie z. B. Natriummethylat und Kalium-tert.-butylat, Alkaliamide, wie z. B. Natriumamid und Alkalihydride, wie z. B. Natriumhydrid, in Frage.

Die Reaktionstemperatur wird bei Verfahren (6) zwischen 0 und 150 °C, vorzugsweise zwischen 10 und 100 °C gehalten. Das Verfahren wird im allgemeinen bei Normaldruck durchgeführt.

Auf 1 Mol 3-Brom-4-fluor-benzylalkohol setzt man 1 bis 1,5 Mol, vorzugsweise 1 bis 1,2 Mol Base und 1 bis 1,5 Mol, vorzugsweise 1 bis 1,2 Mol Benzylhalogenid ein. In einer bevorzugten Ausführungsform von Verfahren (6) wird die Base in einem Verdünnungsmittel vorgelegt und 3-Brom-4-fluor-benzylalkohol dazu gegeben. Nach etwa einer Stunde wird hierzu das Benzylhalogenid tropfenweise gegeben und das Gemisch wird bis zum Reaktionsende, gegebenenfalls bei mäßig erhöhter Temperatur, gerührt. Die Aufarbeitung kann auf übliche Weise durchgeführt werden, beispielsweise indem man das Reaktionsgemisch mit verdünnter Salzsäure versetzt, mit einem mit Wasser nicht mischbaren Lösungsmittel, wie z. B. Diethylether extrahiert, den Etherextrakt trocknet, filtriert und destilliert.

Das Verfahren zur Herstellung von 3-Brom-4-fluor-benzylhalogeniden aus 3-Brom-4-fluor-benzylalkohol (« Verfahren (8) ») wird vorzugsweise unter Verwendung von Verdünnungsmitteln durchgeführt. Als solche kommen vor allem aliphatische und aromatische, gegebenenfalls halogenierte Kohlenwasserstoffe, wie z. B. Pentan, Hexan, Heptan, Cyclohexan, Petrolether, Ligroin, Benzol, Toluol, Xylol, Methylenchlorid, Ethylenchlorid, Chloroform, Tetrachlorkohlenstoff, Chlorbenzol und o-Dichlorbenzol in Frage.

Bei Verfahren (8) zu verwendende Halogenierungsmittel sind z. B. Thionylchlorid, Phosphortrichlorid, Phosphorpentachlorid, Phosphortribromid und Dibrom-triphenylphosphoran.

Die Reaktionstemperatur liegt bei Verfahren (8) zwischen – 10 und 100 °C, vorzugsweise zwischen 0 und 50 °C. Das Verfahren wird im allgemeinen bei Normaldruck durchgeführt.

Zur Durchführung von Verfahren (8) wird 3-Brom-4-fluorbenzylalkohol, gegebenenfalls in einem der oben angegebenen Verdünnungsmittel, vorgelegt und mit wenigstens der äquivalenten Menge eines Halogenierungsmittels tropfenweise versetzt. Nach Ende der Reaktion kann das Produkt durch Destillation in reiner Form isoliert werden. Eine andere Form der Aufarbeitung besteht darin, daß man das Reaktionsgemisch mit Wasser verdünnt, die organische Phase abtrennt, trocknet, filtriert und destilliert.

Das Verfahren zur Herstellung von 3-Brom-4-fluor-benzylalkohol (« Verfahren (10) ») wird vorzugsweise unter Verwendung von Verdünnungsmitteln durchgeführt. Als solche kommen vor allem Alkohole, wie z. B. Methanol, Ethanol, n- und iso-Propanol, n-, iso-, sek.- und tert.-Butanol in Frage. Iso-Propanol wird als Verdünnungsmittel besonders bevorzugt.

Bei Verfahren (10) als Reduktionsmittel zu verwendende Hydridkomplexe der Formel (VI) sind z. B. Lithium-tetrahydridoaluminat (Lithiumalanat) und Natriumtetrahydridoborat (Natriumboranat). Letzteres wird besonders bevorzugt.

Verfahren (10) wird bei Temperaturen zwischen 0 und 50 °C, vorzugsweise zwischen 10 und 40 °C, und gewöhnlich bei Normaldruck durchgeführt.

In einer bevorzugten Ausführungsform von Verfahren (10) wird der Hydridkomplex der Formel (VI) in einem der oben angegebenen Verdünnungsmittel vorgelegt und 3-Brom-4-fluor-benzoylfluorid langsam zugetropft. Das Reaktionsgemisch wird bis zum Ende der Umsetzung gerührt, mit Eiswasser verdünnt und angesäuert. Dann wird mit einem mit Wasser nicht mischbaren Lösungsmittel, wie z. B. mit Methylenchlorid, extrahiert, der Extrakt getrocknet, filtriert und destilliert.

Das als Ausgangsverbindung bei Verfahren (10) zu verwendende 3-Brom-4-fluor-benzoylfluorid ist Gegenstand einer noch nicht zum Stand der Technik gekennzeichneten deutschen Patentanmeldung (vergleiche p 2 915 738/Le A 19 590).

Man erhält diese Verbindung, wenn man 4-Chlor-benzoylchlorid durch Umsetzung mit Kaliumfluorid

in 4-Fluorbenzoylfluorid umwandelt und letzteres zu 3-Brom-4-fluorbenzoylfluorid bromiert gemäß nachstehendem Formelschema :

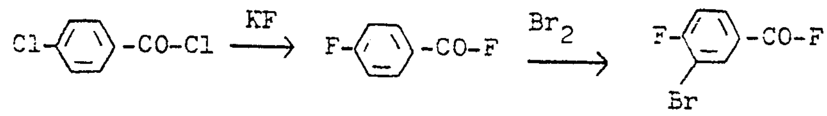


4-Chlor-benzoylchlorid wird mit Kaliumfluorid beispielsweise in Tetramethylensulfon bei Temperaturen zwischen 200 und 220 °C umgesetzt und das Reaktionsgemisch destillativ aufgearbeitet. Man erhält 4-Fluor-benzoylfluorid vom Siedepunkt 53 °C/20 mBar (Brechungsindex : $n_D^{20}$ = 1,479 2).

4-Fluor-benzoylfluorid wird mit elementarem Brom in Gegenwart von 1 % Eisen(III) chlorid bei 70 bis 75 °C umgesetzt. Bei einem Ansatz von 1 Mol erhält man nach Destillation 40 g unverändertes Ausgangsmaterial zurück und 182 g eines Gemisches aus 3-Brom-4-fluor-benzoylfluorid (Siedepunkt : 82-83 °C/15 mBar ; Brechungsindex : $n_D^{20}$ = 1,531 5 ; Schmelzpunkt : 32-34 °C) und 3-Brom-4-fluor-benzoylbromid (Siedepunkt : 123 °C/15 mBar ; Schmelzpunkt : 35-37 °C).

## Beispiel 1

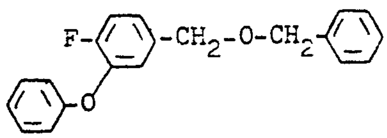

Eine Mischung von 1 Mol (3-Brom-4-fluor-benzyl)-benzylether, 1,1 Mol Natriumphenolat, 0,12 Mol Kupfer(I) oxid, 80 g 4 $MgCO_3 \cdot Mg(OH)_2 \cdot 4H_2O$ und 1 500 ml Isochinolin wird 12 Stunden auf 160 °C erhitzt. Dann wird das Reaktionsgemisch abgekühlt, mit Cyclohexan verdünnt und filtriert. Das Filtrat wird mit verdünnter Salzsäure und Wasser gewaschen, über Natriumsulfat getrocknet, filtriert und destilliert. Man erhält (4-Fluor-3-phenoxybenzyl)-benzyl-ether in einer Ausbeute von 75 % der Theorie in Form eines gelben Öls vom Siedepunkt 135 °C/1 mBar.

Ersetzt man in Beispiel 1 das Magnesiumcarbonat durch 140 g Kaliumcarbonat und verwendet 0,2 Mol Kupfer(I) oxid als Katalysator, so erhält man bei einer Reaktionszeit von 8 Stunden das gleiche Produkt in einer Ausbeute von 70 % der Theorie.

## Beispiel 2

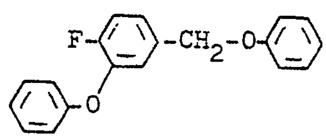

Eine Mischung von 84,3 g (0,3 Mol) (3-Brom-4-fluorbenzyl)-phenyl-ether, 34,8 g (0,3 Mol) Natriumphenolat, 7 g (0,11 Mol) Kupferpulver, 10 g Kaliumchlorid und 50 ml Bis-(2-methoxyethyl)-ether (Diglyme) wird unter Rühren 5 Stunden lang auf 160 °C erhitzt. Der Reaktionsansatz wird dann auf 80 °C abgekühlt, mit 300 ml Toluol versetzt und filtriert. Das Filtrat wird 1 mal mit 100 ml 10 %igem Natriumsulfat gewaschen, anschließend 2 mal mit je 300 ml Wasser ausgeschüttelt. Die organische Phase wird über Magnesiumsulfat getrocknet und das Lösungsmittel im Wasserstrahlvakuum abgezogen. Der ölige Rückstand wird im Vakuum destilliert. Man erhält 70,7 g (80,2 % der Theorie) (4-Fluor-3-phenoxy-benzyl)-phenylether mit dem Siedepunkt : 195-197 °C/3 mBar als sehr zähes Öl.

## Beispiel 3

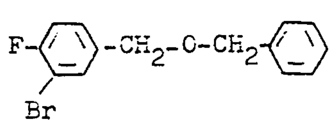

Zu einer Lösung von 12,3 g (0,11 Mol) Kalium-tert.-butylat in 200 ml Tetrahydrofuran gibt man bei 20 °C 20,5 g (0,1 Mol) 3-Brom-4-fluor-benzylalkohol. Dabei steigt die Temperatur auf ca. 40 °C. Man läßt

ca. 1 Stunde bei 20 °C nachrühren und tropft dann 12,6 g (0,1 Mol) Benzylchlorid zum Reaktionsgemisch. Dann wird eine Stunde unter Rückfluß erhitzt. Anschließend wird abgekühlt. Es werden ca. 200 ml Wasser und 50 ml konzentrierter Salzsäure zum Reaktionsgemisch gegeben, und es wird zweimal mit je 200 ml Diethylether extrahiert. Die vereinigten Extrakte werden eingeengt, über Natriumsulfat getrocknet und dann fraktioniert destilliert. Man erhält 14,2 g (48,2 % der Theorie) (3-Brom-4-fluor-benzyl)-benzyl-ether in Form eines hellgelben Öls vom Siedepunkt 165 °C/7 mBar.

Beispiel 4

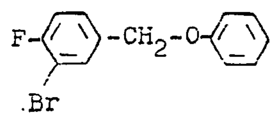

Zu einer Suspension von 5,8 g (0,05 Mol) Natriumphenolat in 100 ml Acetonitril werden bei 20-25 °C unter Rühren 13,4 g (0,05 Mol) 3-Brom-4-fluor-benzylbromid zugetropft. Anschließend wird unter Rühren 3 Stunden lang auf 80 °C erhitzt. Nach dem Abkühlen wird der Reaktionsansatz in 500 ml Wasser gegossen und 2 mal mit je 200 ml Methylenchlorid extrahiert. Die organische Phase wird abgetrennt, über Magnesiumsulfat getrocknet und das Lösungsmittel im Wasserstrahlvakuum abgezogen. Der Rückstand wird im Vakuum destilliert, man erhält 12,6 g (90 % der Theorie) (3-Brom-4-fluor-benzyl)-phenyl-ether mit dem Siedepunkt 145-147 °C/3 mBar.

Beispiel 5

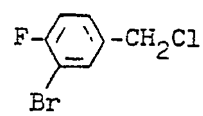

41 g (0,2 Mol) 3-Brom-4-fluor-benzylalkohol werden in 250 ml Tetrachlorkohlenstoff gelöst und bei 20 °C unter Rühren 26 g Thionylchlorid zugetropft. Dann wird 4 Stunden bei 25-30 °C gerührt, anschließend das Lösungsmittel und überschüssiges Thionylchlorid im Wasserstrahlvakuum bei 20 °C abgezogen. Der verbleibende Rückstand wird im Vakuum destilliert. Man erhält 29 g (64,9 % der Theorie) 3-Brom-4-fluor-benzylchlorid mit dem Siedepunkt 85-87 °C/3 mBar.

Beispiel 6

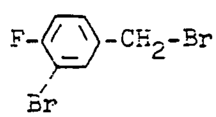

20,5 g (0,1 Mol) 3-Brom-4-fluor-benzylalkohol werden in 100 ml wasserfreiem Toluol gelöst und bei 0 bis 10 °C unter Rühren 10 g Phosphortribromid zugetropft. Dann wird 2 Stunden bei Raumtemperatur gerührt. Anschließend wird der Reaktionsansatz in 500 ml Wasser gegossen, die organische Phase wird abgetrennt, über Magnesiumsulfat getrocknet und das Lösungsmittel im Wasserstrahlvakuum abdestilliert. Der Rückstand wird im Vakuum destilliert. Man erhält 24 g (89,6 % der Theorie) 3-Brom-4-fluor-benzylbromid mit dem Siedepunkt 99-100 °C/3 mBar.

Beispiel 7

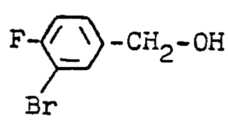

221 g (1 Mol) 3-Brom-4-fluor-benzoylfluorid werden zu einer Mischung von 30,4 g (0,8 Mol) Natriumtetrahydridoborat und 840 ml iso-Propanol bei 20-25 °C innerhalb von 3 Stunden tropfenweise gegeben. Das Reaktionsgemisch wird noch ca. 30 Minuten gerührt und dann in 2 l Eiswasser gegossen. Durch Zugabe von konzentrierter Salzsäure wird pH 1 eingestellt und dann die organische Schicht abgetrennt. Die wässrige Phase wird mit 200 ml Methylenchlorid nachextrahiert. Die vereinigten organischen Phasen werden getrocknet, filtriert und destilliert. Man erhält 163 g (79,5 % der Theorie) 3-Brom-4-fluor-benzylalkohol vom Siedepunkt 137 °C/16 mBar und vom Brechungsindex $n_D^{20}$ = 1,562 3.

Beispiel zur Herstellung von 4-Fluor-3-phenoxy-benzylbromid:

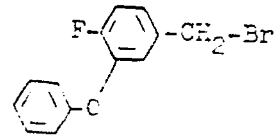

Eine Mischung von 14,7 g (0,05 Mol) (4-Fluor-3-phenoxybenzyl)-phenyl-ether, 50 ml 32 %ige wässrige Bromwasserstoffsäure und 60 ml Eisessig wird unter Rühren 10 Stunden zum Rückfluß erhitzt. Anschließend wird die Reaktionsmischung in 200 ml Wasser gegossen und 2 mal mit je 200 ml Methylenchlorid extrahiert. Die vereinigten Methylenchloridphasen werden anschließend 2 mal mit je 100 ml 10 %iger Natronlauge ausgeschüttelt. Anschließend wird die organische Phase über Magnesiumsulfat getrocknet und dann das Lösungsmittel im Wasserstrahlvakuum abgezogen. Der verbleibende Rückstand wird durch Andestillieren bei 60 °C Badtemperatur/3 mBar von den letzten Lösungsmittelresten befreit. Man erhält so das 4-Fluor-3-phenoxy-benzylbromid als zähes Öl.

**Ansprüche**

1. 4-Fluor-3-phenoxy-benzyl-ether der Formel I

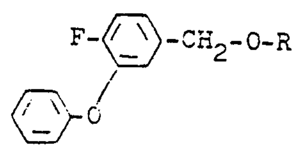

(I)

in welcher R für Phenyl oder Benzyl steht.

2. Verfahren zur Herstellung der neuen Verbindungen der Formel (I), dadurch gekennzeichnet, daß man 3-Brom-4-fluor-benzyl-ether der Formel II

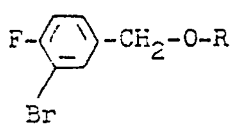

(II)

in welcher R die oben angegebene Bedeutung hat, mit Alkali- oder Erdalkali-phenolaten, gegebenenfalls in Gegenwart von Hilfsstoffen aus der Reihe der Alkali- oder Erdalkali-halogenide oder -carbonate und in Gegenwart von Kupfer oder Kupferverbindungen als Katalysatoren sowie unter Verwendung von Verdünnungsmitteln bei Temperaturen zwischen 100 und 200 °C umsetzt.

3. Verwendung der neuen Verbindungen der Formel (I) als Zwischenprodukte zur Herstellung von 4-Fluor-3-phenoxy-benzyl-bromid durch Umsetzung mit Bromwasserstoff nach bekannten Etherspaltungsmethoden.

**Claims**

1. 4-Fluoro-3-phenoxy-benzyl ethers of the formula I

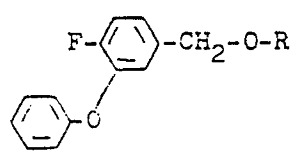

(I)

in which R represents phenyl or benzyl.

2. Process for the preparation of the new compounds of the formula (I), characterised in that 3-bromo-4-fluoro-benzyl ethers of the formula II

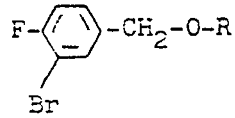

(II)

in which R has the meaning indicated above, are reacted with alkali metal phenolates or alkaline earth metal phenolates, if appropriate in the presence of auxiliaries from the series comprising alkali metal halides or carbonates and alkaline earth metal halides or carbonates, and in the presence of copper or copper compounds as catalysts, and using diluents, at temperatures between 100 and 200 °C.

3. Use of the new compounds of the formula (I) as intermediate products for the preparation of 4-fluoro-3-phenoxy-benzyl bromide by reaction with hydrogen bromide by known methods for splitting ethers.

**Revendications**

1. Ether de 4-fluoro-3-phénoxybenzyle de formule

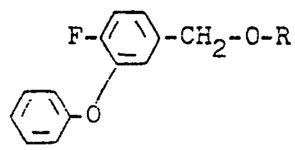

(I)

dans laquelle R est le groupe phényle ou benzyle.

2. Procédé de production des composés nouveaux de formule I, caractérisé en ce qu'on fait réagir un éther de 3-bromo-4-fluorobenzyle de formule II

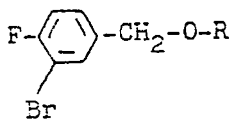

(II)

dans laquelle R a la définition indiquée ci-dessus, avec des phénolates de métaux alcalins ou alcalino-terreux, éventuellement en présence de substances auxiliaires de la série des halogénures ou carbonates de métaux alcalins ou alcalino-terreux et en présence de cuivre ou de composés de cuivre comme catalyseurs, de même qu'en utilisant des diluants, à des températures comprises entre 100 et 200 °C.

3. Utilisation des nouveaux composés de formule (I) comme produits intermédiaires pour l'obtention de bromure de 4-fluoro-3-phénoxybenzyle par réaction avec le bromure d'hydrogène par des procédés connus de clivage d'un éther.